(19) Europäisches Patentamt
European Patent Office
Office européen des brevets

(11) Publication number: 0 402 020 A1

(12) **EUROPEAN PATENT APPLICATION**

(21) Application number: 90305738.8

(22) Date of filing: 25.05.90

(51) Int. Cl.5: C07C 265/14, C07C 263/06, C07C 275/28, C07C 273/18

(30) Priority: 05.06.89 US 361467

(43) Date of publication of application: 12.12.90 Bulletin 90/50

(84) Designated Contracting States: BE DE FR GB IT NL

(71) Applicant: ARCO CHEMICAL TECHNOLOGY INC.
3 Christina Centre Suite 902 201 N Walnut Street
Wilmington Delaware 19801(US)

(72) Inventor: Shawl, Edward T.
208 Martroy Lane
Wallingford, Pennsylvania 19086(US)
Inventor: Kesling, Haven S., Jr.
248 Friendship Road
Drexel Hill, Pennsylvania 19026(US)
Inventor: Liotta, Frank J. Jr.
105 Larchwood Court
Collegeville, Pennsylvania 19426(US)
Inventor: Zajacek, John G.
669 Clovelly Road
Devon, Pennsylvania 19333(US)

(74) Representative: Cropp, John Anthony David et al
MATHYS & SQUIRE 10 Fleet Street
London, EC4Y 1AY(GB)

(54) **An integrated process for the preparation of methylene diphenylene diisocyanates and polymethylene polyphenylene poly (isocyanates) from diamines and polyamines.**

(57) A multi-step process for the preparation of methylene diphenylene diisocyanates (MDI) or polymethylene polyphenylene poly (isocyanates) (PMDI) from methylene diphenylene diamines or polymethylene polyphenylene polyamines comprises reacting the diamine or polyamine with isocyanic acid to form a urea which is then reacted with a dialkyl amine to give a corresponding dialkyl urea or poly (alkyl urea) product which is then thermally reacted in the presence of various reaction promoters to give the corresponding isocyanate MDI or PMDI product.

EP 0 402 020 A1

# AN INTEGRATED PROCESS FOR THE PREPARATION OF METHYLENE DIPHENYLENE DIISOCYANATES AND POLYMETHYLENE POLYPHENYLENE POLY (ISOCYANATES) FROM DIAMINES AND POLYAMINES

## FIELD OF THE INVENTION

The present invention relates to a multi-step process for the preparation of methylene diphenylene diisocyanates and the higher polymethylene polyphenylene poly (isocyanate) homologs thereof (commonly known in the trade as MDI and PMDI respectively) from methylene diphenylene diamines or polymethylene polyphenylene polyamines and isocyanic acid to form a methylene diphenylene bis urea or a polymethylene polyphenylene polyurea respectively, which urea is then reacted with a dialkyl amine such as diethylamine, to give the methylene diphenylene (dialkyl urea) or polymethylene polyphenylene poly (alkylurea) products which are then thermally treated in a inert organic solvent in the presence of a reaction promoter to produce the corresponding isocyanates which, among other things are especially useful for the preparation of polyurethanes and modified polyurethanes.

## BACKGROUND OF THE INVENTION

A number of processes have been reported for the preparation of mono- and disubstituted ureas and amines as in the preparation of, for example, the bis (diethylurea of toluene by reacting toluene -2,4-diisocyanate with diethylamine described in French Patent No. 1570670, June 13, 1969.

An article by N.A. Ivanov entitled "Synthesis of Substituted Ureas and Thioureas and Their Thermal Stability", Chemistry Department of the Kalinin Agricultural Institute describes the synthesis of thioureas, mono substituted ureas and toluene-2, 4-diamines.

German Democratic Republic Industrial Patent No. 228,544 related to the production of acyl isocyanates describes the synthesis of 1, 1-diacyl-3, 3-dialkylureas from, for example 1, 1-dimethylurea.

Czechoslovakian Patent No. 200,441 discloses a method for the preparation of aminophenylurea by reacting phenylenediamine diamine with one mole of cyanic acid in the presence of sodium or potassium cyanate.

An article of Y. Shimonura et al entitled "Reactions of Isocyanic Acid with Various Reagents", Fukui Daigaku Kogakuba Kenkyu Hokoku, Vol. 31, No. 2, pp 115, 1983 describes the reaction of 2-cyanoethylamine with isocyanic acid to give 2-cyanoethylurea. The synthesis of isocyanic acid from cyanuric acid by thermal decomposition is also set forth.

Applicants have found that tertiary amine hydrohalides, such as pyridine hydrochloride, phosphorus pentoxide ($P_2O_5$) organic sulfonic acids and sulfonated aromatic ion exchange resins are all excellent promoters for the thermal decomposition of the intermediate dialkyl ureas or poly (alkylureas) of this invention to the corresponding isocyanate and at relatively mild reaction temperatures and short residence times in an organic solvent.

Processes have been reported in the literature for the preparation of aromatic mono- and polyisocyanates by the vapor or solvent phase decomposition of substituted ureas using various promoters to help convert the urea groups to isocyanate groups.

The vapor phase production of aromatic isocyanates from symmetrical bis aryl ureas in a solventless system in the presence of hydrogen chloride,phosphorus pentoxide or zinc chloride was described by A. Hofmann in the Proc. Royal Soc., London, Vol. IX p. 274 (1858). By heating a mixture of diphenyl urea with phosphorus pentoxide, zinc chloride or gaseous HCl. Hofmann distilled phenyl isocyanate ovehead. No details of the experimental procecure are presented and yield of isocyanate not given.

A. Hofmann, Chemisch Berichte, Vol. 3, pp. 653-658 (1870) described heating diphenyl urea in the presence of phosphoric acid giving yields too small to be considered for the preparation of the isocyanate.

Subsequent work by Iwakura and Nagakubo reported in the Bulletin Tokyo Inst. Technol., Vol. 13, p.25 (1950) and Chemical Abstracts, Vol.44, p. 3924e (1950) describes the preparation of an aromatic isocyanate (p-ethoxyphenylisocyanat) by heating a solution of bis aryl urea such as bis (p-ethoxyphenyl) urea in the presence of hydrogen chloride gas.

The vapor phase decomposition of bis aryl ureas at 350°C° and higher temperatures has been described by W.D. Bennet et al, Journ. Am. Chem. Soc., Vol. 75, p.2101 (1952) and Slocombe et al in U.S. Pat. No. 2,773,086, December 4, 1956 in the presence of gaseous HCl as a promoter. Yields are reported in the 60 to 70% range for the vapor phase reaction and only a 5% yield for liquid phase reaction. A carbamoyl chloride intermediate is formed.

The liquid phase decomposition of trisubstituted ureas to isocyanates has been described by van Landeghem et al, French Patent No. 1,473,821, February 13, 1967; C.J. Hearsey, U. S. Patent No. 3,898,259, August 5, 1975 and Rosenthal et al in the U.S. Patent No. 3,936,484, February 3, 1976. van Landeghem shows thermal decomposition of trisubstituted ureas in an organic solvent having specified dielectric constants at 140° to 170°C with long reaction times of from 6 to 10 hours and modest yields of 60 to 75%. A variety of catalysts are shown but not exemplified or claimed, and include metal salts, such as acetates, stearates, and linoleates of manganese, zinc, cobalt, chromium and vanadium, tertiary amine bases, such as aliphatic, cycloaliphatic, aromatic and mixed tertiary amines, aliphatic heterocyclic amines such as N-methylpiperidine or N, N′-dimethylpiperidine as well as aromatic heterocyclic amines such as pyridine and pyrimidine. Other nitrogen compounds such as imidazole are indicated as being suitable. However, under the reaction conditions described, tertiary amines as shown by van Landeghem do not catalyze urea decomposition.

Rosenthal et al U.S. Patent No. 3,936,484 discloses the thermal decomposition of di- and tri-substituted ureas to isocyanates at temperatures above 230°C in a solvent with short residence times and isocyanate yields of from 60 to 80%.

The Hearsey U.S. Patent No. 3,898,259 describes the introduction of gaseous hydrogen chloride into the liquid phase urea decomposition reaction to give reduced reaction times with isocyanate yields of from 80 - 90%. An excess of gaseous HCl is employed at temperatures of from 100° to 200°C and a by-product carbamoyl chloride intermediate formed.

A.Hentschel et al U.S. Patent No. 4,223,145, September 16, 1980 discloses the formation of an HCl adduct of a tri-substituted urea using at most, a 10% excess of HCl. This adduct is then decomposed in a closed system at from 80° to 180° C.

## SUMMARY OF THE INVENTION

This invention relates to a novel integrated multi-step process for the preparation of methylene diphenylene diisocyanates and the higher polymethylene polyphenylene poly (isocyanate) homologs thereof from diamines or polyamines and isocyanic acid (HNCO). The methylene diphenylene diamines or or polymethylene polyphenylene polyamines are reacted with isocyanic acid to convert the amino groups to urea groups, which methylene diphenylene bis ureas or polymethylene polyphenylene poly (ureas) are then reacted with a dialkyl amine, such as diethylamine to produce a methylene diphenylene (dialkyl urea) or polymethylene polyphenylene poly (alkyl urea) product which is slurried in or dissolved in an inert organic solvent and thermally decomposed in the presence of a reaction promoter selected from tertiary amine hydrohalides, phosphorus pentoxide, organic sulfonic acids and sulfonated aromatic ion exchange resins to produce the corresponding isocyanate.

It is an object of the present invention therefore, to provide an improved multi-step process for the preparation of diisocyanates and polyisocyanates (MDI and PMDI) from the urea reaction product of a diamine or polyamine and isocyanic acid which has been reacted with a dialkyl amine to give a methylene diphenylene (dialkyl urea) or polymethylene polyphenylene poly (alkyl urea) precursor to the corresponding isocyanate.

It is another object of this invention to provide an improved reaction (thermal decompostion) system for the conversion of the intermediate dialkyl or polyalkyl ureas to the corresponding isocyanate product.

These and other objects and advantages of this invention will become apparent from the description of the invention which follows, and from the claims.

## DETAILED DESCRIPTION OF THE INVENTION

In accordance with the present invention a methylene diphenylene diisocyanate or polymethylene polyphenylene poly (isocyanate) is produced by a process which comprises the steps of:

(a) reacting a methylene diphenylene diamine having the formula:

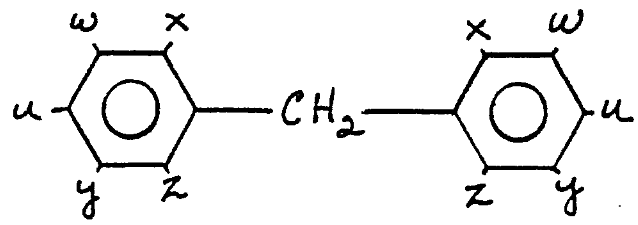

or a polymethylene polyphenylene polyamine having the formula

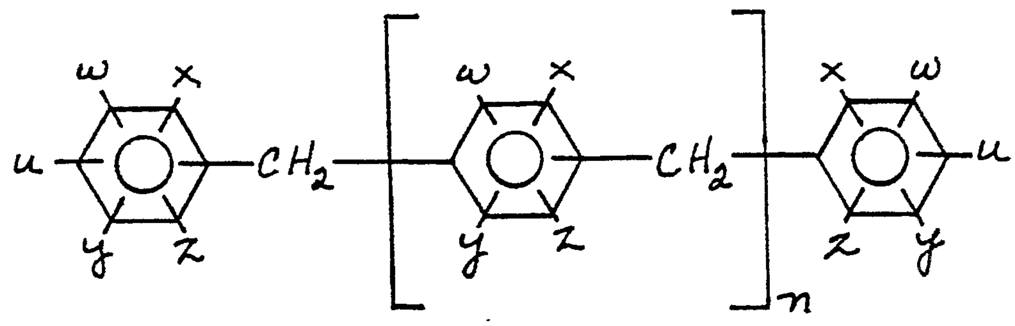

wherein n equals an integer of from 1 to 8 and at least one of the substituents u, w, x, y and z on the ring is a amino group and the other substituents which may be different on the ring, are hydrogen, a 1 to 6 carbon alkyl group, a halogen, ether or nitro group, with isocyanic acid at a temperature of from about -30 °C to about 200 °C preferably from about -10 °C to about 150 °C in the presence of a solvent or mixture of solvents which are stable and substantial chemically inert to the components of the reaction system, to convert the amino groups of the methylene diphenylene diamine or polymethylene polyphenylene polyamine to urea groups ($-NHCONH_2$) to produce a corresponding methylene diphenylene bis urea or a polymethylene polyphenylene polyurea:

(b) reacting said bis urea or polyurea thus produced with a dialkyl amine ($NHR'R''$) wherein $R'$ and $R''$ are substituted or unsubstituted mono-valent radicals selected from saturated or mono-olefinic unsaturated straight or branched chain aliphatic or cycloaliphatic radicals optionally containing alkoxyalkyl radicals with one or more ether linkages, aryl radicals, or aralkyl radicals, or $R'$ and $R''$ together form a substituted or unsubstituted saturated or mono-olefinic unsaturated divalent aliphatic radical, at a temperature of from about 50 °C to about 200 °C, preferably from about 90 °C to 150 °C in the presence of a solvent or mixture of solvents which are stable and substantially chemically inert to the components of the reaction system to produce a methylene diphenylene bis (dialkyl urea) having the general formula:

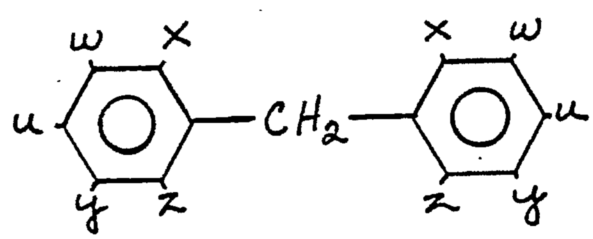

or the polymethylene polyphenylene poly (alkyl urea) homologs thereof having the following structural formula:

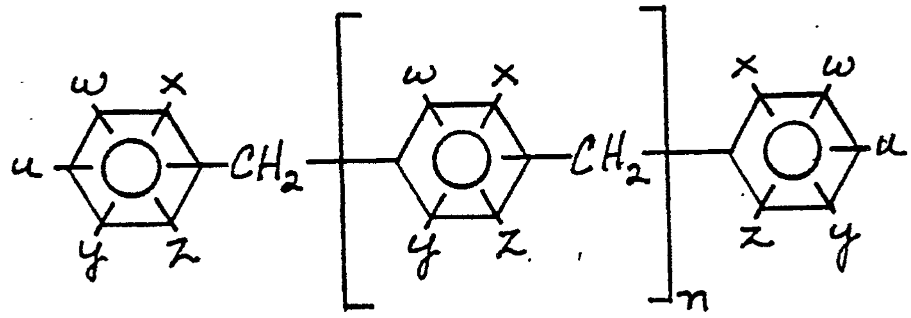

wherein n equals an integer of from 1 to 8 and at least one of the substituents u, w, y and z on the ring is a dialkylureido group(-NHCONR$'$R$''$) and the other substituents which may be different on the ring, are hydrogen, a 1 to 6 carbon alkyl group, a halogen, an ether group or a nitro group, and R$'$ and R$''$ are as defined above;

(c) thermally decomposing said methylene diphenylene bis (dialkylurea) or polymethylene polyphenylene poly (alkylurea) dissolved in or slurried in an organic solvent or mixture of solvents which are stable and substantially chemically inert to the.components of the reaction system, in the presence of a reaction promoter selected from the group consisting, of a tertiary amine hydrohalide, such as pyridine hydrochloride, phosphorus pentoxide, organic sulfonic acids, and sulfonated aromatic ion exchange resins, at a temperature within the range of from about 50 °C to about 220 °C, preferably from about 90 °C to about 150 °C; and

(d) recovering the methylene diphenylene diisocyanate or polymethylene polyphenylene poly (isocyanate).

The isocyanic acid employed in Step (a) of the process of the present invention may be produced or generated by known methods such as the pyrolysis of urea or cyanuric acid, reaction of cyanate salts such as sodium, potassium or silver cyanate and the like with an acid such as acetic or hydrochloric acid and the like. The isocyanic acid may be generated and used in situ, or it may be distilled away from its source and used in the process of the invention as a gas or dissolved in an appropriate solvent.

In the present process the molar ratio of the ($-NH_2$) groups of the diamines or polyamines to isocyanic acid is generally one to one. However, an excess of isocyanic acid of up to about 50% may be employed. Alternatively, an excess of up to about 30% ($-NH_2$) groups may be used and any unreacted or partially reacted diamines or polyamines separated from the bis urea produced and recycled. In the subsequent reaction employing the bis urea the molar ratio of the dialkylamine reactant, such as dimethylamine, to the urea groups ($-NHCONH_2$) is generally one to one. However, an excess of dialkylamine of from about 10% to a ten-fold excess may advantageously be employed to drive the reaction to completion. Unreacted dialkylamine may be easily recovered by distillation for recycle in the reaction.

The dialkylamines or mixtures thereof which may be employed in Step (b) of the process of the invention conform to the general fomula R$'$R$''$HN wherein R$'$ and R$''$ are as defined above. Preferably R$'$ and R$''$ are each alkyl groups having from 1 to 8 carbon atoms. Representative dialkylamines include, for example, dimethylamine, diethylamine, methylethylamine, diisopropylamine, dicyclohexylamine, dibutylamine, cyclopentylamine, cyclohexylamine, bis (2-methoxyethyl)amine, bis (2-phenoxyethyl)mine, and the like.

Solvents or mixtures of solvents which are stable and substantially chemically inert to the components of the reaction system are employed in each of the process steps of the present invention. Suitable solvents which may be employed include, for example, the aromatic hydrocarbons such as benzene, toluene, xylene, tetrahydronaphthalene as well as higher alkyl-substituted aromatic hydrocarbons; alkanes and substituted alkanes as well as cycloalkanes having from 5 to 20 carbon atoms such as, for example, n-hexane, n-heptane, octane, nonane, cyclohexane, dodecane, octadecane, 2-methyl- hexane, 2-ethylhexane, methylcyclohexane, cyclopentane and the like; halogenated or nitrated aromatic or aliphatic hydro- carbons such as, for example, methylene chloride, chloroform, carbontetrachloride, 1 ,2-dichloroethane, chlorobenzene, trichloroethane, tetrachloroethane dichlorobenzene, nitrobenzene dinitrotoluenes and the like; aromatic or aliphatic ether such as for example, diphenylether and dibutylether and the like; tertiary amines such as, for example, pyridine, triethylamine, N-methylpyrolidone and the like. Certain ketones, esters, alcohols as well as water and highly polar solvents, such as sulfolane, dimethysulfoxide, ethylene carbonate or propylene carbonate may also be used. It is not necessary that the diamines, polyamines, the reaction intermediates such as the bis ureas, polyureas or the reaction products be completely miscible with the

solvents at the concentrations employed. Advantage may be taken of differing solubilities of the reagents, intermediates and reaction products in the various solvents or mixture of solvents to separate the reaction components or to drive the reaction to increased product. The same solvent or mixture of solvents may be used throughout the reaction system or different solvents or solvent mixtures used in different steps of the process.

The intermediate bis urea or polyurea obtained by the reaction of the diamine or polyamine with isocyanic acid in in Step (a) may, if desirable be isolated from the reaction system and further processed according to the process of the invention or it may simply be carried forward as a solution or a slurry without isolation for completion of the process to produce the desired isocyanate. In appropriate solvents, the intermediate bis urea or polyurea may be essentially insoluble and the urea easily separated from unreacted diamine or polyamine or partly reacted amine urea by-products by conventional techniques such as filtration, centrifugation, and the like. The separated bis urea,polyurea or the crude intermediate product may be reacted with a dialkyl amine to produce the desired bis dialkyl urea or poly (alkyl urea). The dialkyl amine reactant may be added as a gas, a liquid, a solid or as a solution in solvent. Alternatively, the dialkyl amine may be added to the reactor along with the diamine or polyamine. Ammonia generated in this part of the reaction is removed by any convenient means.

The $R'$ and $R''$ of the dialkylureido (-NHCONR'R'') group described hereinabove may also be substituted or unsubstituted monovalent radicals selected from saturated or mono-olefinic unsaturated straight or branched chain aliphatic or cycloaliphatic radicals optionally containing alkoxyalkyl radicals with one or more ether linkages, aryl radicals, or aralkyl radicals or R and $R'$ together form a substituted or unsubstituted saturated or mono-olefinic unsaturated divalent aliphatic radical.

Representative methylene diphenylene bis (dialkylureas) produced as intermediate products of the present invention include, for example, methylene diphenylene bis (dimethylurea), methylene diphenylene bis (diphenyl or diethyl or dibutyl ureas) and the like as well as the polymethylene polyphenylene poly (diethyl or dibutyl or dipropyl, etc. ureas).

The tertiary amine hydrohalide salts employed in the process of the present invention to promote thermal decomposition of the bis dialkyl or polyalkyl ureas to the corresponding isocyanate may be prepared, for example, by reacting the tertiary amine selected with a hydrogen halide such as HCl. Salts of hydrogen fluoride, chloride, bromide or iodide may be used. The tertiary amines used to prepare the hydrohalide will conform to the general formula R,R',R''N wherein R,R' and R'' are not hydrogen but may be an aliphatic radical having from 1 to 10 carbon atoms, a cycloatiphatic radical such as cyclopentyl, cyclohexyl and cycloheptyl radicals, an aromatic radical, or an aralkyl radical. Such radicals may be substituted with, for example, nitro or halo groups which are non-reactive with the isocyanate produced. Suitable amine salts include, for example, triethylamine hydrochloride, hydrobromide or hydrofluoride, trioctylamine hydro-chloride, hydrobromide or hydrofluoride, N-Methyldiethylamine hydrobromide or hydrochloride, N,N-diethylaniline hydrochloride and N,N-dimethylcyclohexylamine hydrochloride. Hydrohalide salts of heterocyclic tertiary amines and heterocyclic aromatic amines may also be employed. Representative salts include, for example, N-methylpyrrolidine hydrochloride, pyridine hydro- chloride or hydrobromide, 3-ethylpyridine hydrochloride, the hydrohalide salt of 1,4- diazabicyclo [2.2.2] octane, 4-chloro- pyridine hydrochloride, 4,4'-bipyridine dihydrochloride, quinoline hydrochloride or the like. Salts of amine oxides such as 2-chloropyridine N-oxide hydrochloride may also be used as a promoter. In addition, the hydrohalide may be formed with an amine which may be part of a polymer such as polyvinyl pyridine or a resin prepared from tertiary amine groups attached to a styrene divinylbenzenepolymer. The tertiary amine hydrohalide is generally employed in the process at a molar ratio of one to one based on the urea groups. However, an excess of the tertiary amine hydrohalide promoter may be used.

The phosphorus pentoxide employed in the process of the present invention to promote thermal decomposition of the bis dialkyl or polyalkyl ureas to the corresponding isocyanate is commercially available material produced by burning phosporus in dry air. The phosphorus pentoxide promoter is generally employed in the process at a molar ratio of one to one based on the urea groups. However, an excess of the phosphorus pentoxide promoter of up to three to one may be used. No advantage is gained by using higher amounts which may lead to by-product formation.

The organic sulfonic acid employed in the process of the present invention to promote the thermal decomposition of the bis dialkyl or polyalkyl ureas may be an alkane sulfonic acid or a halogenated alkane sulfonic acid having up to 10 carbon atoms in the alkyl group, or an aromatic sulfonic acid which may contain substituents on the aromatic ring such as halogens, alkyl radicals, aromatic radicals, nitro groups and the like. The organic sulfonic acid may be in the form of an acidic sulfonated aromatic ion exchange resin such as, for example, the sulfonated styrene/divinyl-benzene copolymer (sold, for example, commercially as "Amberlyst 15" by Rohm & Haas Co.) and having a bulk density of approximately 565 g./l, a

hydrogen ion concentration of approximately 4.9 milliequivalents/g.dry, a surface area of from about 40 to 50 $m^2$/g. and an average pore diameter of from about 200 to 600 Angstrom units, or an acidic perfluoroalkane sulfonic acid resin such as "Nafion" (sold for example, commercially by the DuPont Co.) and having an equivalent weight of between about 110 and 1500, a hydrogen ion concentration of between about 0.7 - 1.0 milliequivalents/g. dry and prepared, for example, by the polymerization of tetrafluoroethylene with a sulfonyl fluoride vinyl ether, followed by saponification with caustic to form the alkali metal salt and treatment with an acid to convert the salt to the sulfonic acid form. Mixtures of the sulfonic acid promoters may be employed but it is preferable to use a single acid promoter to simplify separation and recovery of the aromatic isocyanate produced. Representative organic sulfonic acid promoters suitable for use in the process of this invention include, for example, methane, ethane, butane, hexane sulfonic acids, and the like, trifluoromethane sulfonic acid, benzene sulfonic acid, 1,3-benzene disulfonic acid, p-toluene sulfonic acid, naphthalene sulfonic acid, 4-chloro-3-nitrobenzene sulfonic acid, 3 nitrobenzene sulfonic acid, and the like, as well as the sulfonated aromatic ion exchange resins which include the "Amberlyst 15" and "Nafion" described hereinabove and also include the "Dowex 50" (Dow Chemical), "AG50W" (Bio-Rad), and "Amberlite" (Rohm and Haas) resin materials. The ion exchange resins may be supplied commercially in the hydrogen ion form or the salt form such as the sodium or potassium salt. The salt can readily be converted to the active hydrogen ion form by, for example, treating with aqueous hydrochloric acid, washing with water to a constant pH in the range of 5.5 to 7 and then drying to remove residual water.

The organic sulfonic acid promoter is generally employed in the process of the instant invention at a molar ratio of one to one based on the urea groups to sulfonic acid groups. No advantage is gained by using large excess amounts which may lead to by-product formation.

The process of the present invention can be suitably carried out by adding the bis dialkyl or polyalkyl urea to a solvent or a mixture of solvents comprising the reaction medium. The urea may be soluble in the solvent or solvents or soluble at reaction temperatures or the urea may be in the form of a slurry. Reactions using a sulfonic acid ion exchange resin promoter may also be carried out in a fixed bed of resin by passing a solvent solution of the urea through a bed maintained at the desired reaction temperature. A general procedure for carrying out Step (a) of the process is to charge the methylene diphenylene diamine or polyamine together with a solvent into the reaction vessel. The isocyanic acid is introduced into the reactor as a gas, optionally diluted with an inert gas, as a liquid, or as a solution in an appropriate solvent. Alternatively, the isocyanic acid can be charged to the reactor first together with a solvent and the diamine or polyamine then added as a liquid, a solid, a solution in suitable solvent or as a slurry in a suitable inert liquid. The reaction vessel is heated or cooled as necessary to provide the desired reaction temperature for the appropriate period. Heating and/or cooling means may be employed interior or exterior of the reaction vessel to maintain temperature within desired ranges. The desired reaction product may be recovered by standard filtration and/or distillation procedures.

In the process of the present invention, the Step (a) reaction of the diamines or polyamines with isocyanic acid will proceed at temperatures of from about -30°C to about 200°C, preferably from about -10°C to about 100°C. Reaction time is dependent on the temperature but will generally range between about two minutes to several hours. The Step (b) reaction of the intermediate bis urea or polyurea and the dialkyl amines will proceed at temperatures of from about 50°C to about 200°C, preferably from about 90°C to about 150°C. The reaction time depends on the temperature but will generally range between about 30 minutes to about 8 hours.

The process of the present invention may be carried out as a batch, semi-continuous or continuous process and the order of addition of the materials and reactants may be varied to suit the particular apparatus, reactants and promoter employed.

The reaction of the invention may be carried out in any suitable reactor which is equipped with a means for temperature control and agitation. Heating and/or cooling means may be employed interior or exterior of the reaction vessel to maintain temperature within the desired range.

As indicated hereinabove, the Step (c) thermal decomposition of the bis dialkyl or poly (alkyl) ureas is carried out at temperatures of from about 50°C to about 220°C, preferably from about 90°C to 150°C. Reaction time is dependent on decomposition temperature but will generally range between about 5 minutes and several hours.

The reaction steps of this invention are generally carried out at atmospheric pressure, but depending on the boiling points of the solvents employed and the isocyanate product, it may be carried out at super-atmospheric or sub-atmospheric pressures. The isocyanate formed may be recovered by filtration, by distillation of either the solvent or the isocyanate, whichever is lower boiling, or by other known methods, and will depend on the solvent employed and the isocyanate produced.

The present invention is more fully illustrated by the following examples, which include particular features of the invention. However, the examples are not to be construed as limiting the invention in any way, it being understood that numerous variations are possible without departing from the spirit and scope of the invention.

## EXAMPLE 1

A mixture of 2.0g (10 mmoles) of 4,4′-methylene diphenylene diamine in 130g o-xylene was charged to a 250 ml, 3 neck round bottom flask equipped with a mechanical stirrer, condenser and thermometer. This mixture was heated to 130 °C and the resulting solution was stirred while 15g of 7.0 weight percent solution of isocyanic acid in o-xylene containing 1.05g (24 mmoles ) of isocyanic acid was added over 10 minutes. Solids began to precipitate immediately on addition of the isocyanic acid solution. After 20 min. additional mixing, the solids were collected by filtration. Analysis of the filtrate and the recovered solids by high pressure liquid chromatography (HPLC) showed 96% conversion of the methylene diphenylene diamine with 98% selectivity to methylene diphenylene bis urea. The solids were returned to the reaction flask, suspended in 100g fresh o-xylene and 2.5g diethylamine (34 mmoles) was added and the mixture was heated for 2 hours. The initial temperature was 100 °C but as the diethylamine was consumed, the temperature rose to 135 °C and the solids dissolved. Analysis of the product by HPLC showed essentially quantitative conversion of the bis urea with 90% selectivity to methylene diphenylene bis (diethylurea). Unreacted diethylamine was distilled out of the reactor and then pyridine hydrochloride, 2.90g (25.1 mmoles) was added to the mixture of 4,4′-methylene diphenylene bis (diethylurea) in o-xylene and the mixture was heated to reflux at 142 °C for 60 min. A sample was taken and reacted with ethanol to convert isocyanate groups to ethyl carbamate groups. This sample was then analyzed by high pressure liquid chromatography (HPLC). Conversion of the 4,4′-methylene diphenylene bis (diethylurea) was 98% with a selectivity of 93% to 4,4′-methylene diphenylene diisocyanate and 5% to the mono-diethylurea mono-isocyanate derivative. The overall yield of 4,4′-methylene diphenylene diisocyanate was 84% based on the starting 4,4′-methylene diphenylene diamine.

## EXAMPLE 2

A 250 ml, 3 neck, round bottom flask equipped with a mechanical stirrer, condenser and thermometer was used. Polymethylene polyphenylene polyamine, 2g, made by condensing aniline with formaldehyde using methods described in the literature (Kirk-Othmer, Encyclopedia of Chemical Technology, 3rd Edition Vol.2, pp 342-343, John Wiley and Sons, N.Y. 1978) in 100g mixed xylenes was treated with 25g of 5.0 weight percent solution of isocyanic acid in mixed xylenes at 80 °C. An oil formed as the isocyanic acid was added. After 30 min. mixing, the excess isocyanic acid was distilled out and then 3g of diethylamine was added. The mixture was heated from an initial temperature of 80 °C to a maximum of 140 °C over three hours. Analysis of the product by a combination of HPLC and nuclear magnetic resonance showed that a mixture of poly methylene polyphenylene poly (diethylureas) had been made with an approximate yield of 85% based on starting polyamine. Unreacted diethylamine was distilled out of the reactor and then methanesulfonic acid, 2.0g, and 50g mixed xylenes were added. The mixture was heated to reflux at 144 °C for 30 minutes and then the xylene phase was analyzed by infrared spectroscopy for the isocyanate group. The yield of polymethylene polyphenylene polyisocyanate was 81%.

## EXAMPLE 3

Isocyanic acid, generated by the reaction of sodium cyanate with hydrogen chloride, was distilled away from the sodium cyanate and carried as a gas diluted with nitrogen into a 250ml, 3 neck round bottom flask equipped with a magnetic stirrer, condenser, and gas inlet tube containing 2.0g of 4,4′-methylene diphenylene diamine in 130g o-dichlorobenzene. The reaction was held at -10 °C by a refrigerated bath. Solids precipitated in the flask on addition of the isocyanic acid. After an excess of isocyanic acid has been added, the reaction was warmed to room temperature and stirred for 30 minutes. The solids formed were

collected by filtration, transferred to a pressure vessel, and suspended in 100 ml mixed xylenes. 3g dimethylamine was charged to the vessel which was then heated to 140° C under autogenous pressure for 4 hours. Analysis of the product by HPLC showed an overall yield of 90% of 4,4′-methylene diphenylene bis (dimethylurea). The product was returned to the round bottom flask and the residual dimethylamine was distilled overhead. Then 3.0g of phosphorus pentoxide was added in one portion and the mixture was heated at 120° C for 90 min. The product characterized by infrared and HPLC analysis, showed 95% conversion of the 4,4′-methylene diphenylene bis (dimethylurea) with a 90% yield of the corresponding isocyanate.

## EXAMPLE 4

Using the procedures of Example 1, a solution of 4,4′-methylene diphenylene bis (diethylurea) was prepared by reacting a mixture of 4.0g, (20 mmoles) of 4,4′-methylene diphenylene diamine in 100g chlorobenzene with 35g of a 5wt% chlorobenzene solution of isocyanic acid, containing 1.75g, (41 mmoles) of isocyanic acid at 50° C for 30 min., adding 4.4g of diethylamine and heating the resulting mixture at reflux for 4 hrs. The product solution was combined with 20g of "Amberlyst 15" resin stirred, and heated for 2 hours at 130° C. At the end of the reaction time, the mixture was cooled to room temperature and solid resin was removed by filtration. Analysis of the chlorobenzene solution by HPLC after addition of ethanol to convert the isocyanate to its ethyl carbamate derivative showed an 85% yield of methylene diphenylene diisocyanate.

## EXAMPLES 5 to 9

A number of runs were carried out according to procedures of Example 1 employing various diamines or polyamines, dialkylamines, solvents, reaction temperatures and cracking promoters. The products were characterized by infrared spectroscopy or high pressure liquid chromatography. The reaction conditions for synthesis of the corresponding bis (dialkyl) urea compounds are set forth in Table 1 below. These urea compounds, including solvent were converted to the corresponding isocyanate. The reaction conditions and results for making the corresponding isocyanates from the bis (dialkylurea) compounds of Table 1 are set forth in Table 2 below.

TABLE 1

| Example No. | Polyamine or Diamine (g.) | Solvent (g.) | HNCO-g. Solvent (g) | Temp. (°C) | Time | Dialkyl Amine (g.) | Temp. (°C) | Time |
|---|---|---|---|---|---|---|---|---|
| 5 | 4′,4′/2,4′ MDA 90.10 (1.22) | Diphenyl Ether (100) | 1.00 (as gas) | 150 | 10 min. | DEA (15) | 150 | 2 hrs |
| 6 | PMPPA (2.4) | Nitro-Benzene (150) | 5 (as gas) | 30 | 1 hr. | DMA (15) | 140 | 2 hrs |
| 7 | 4,4′-MDA (1.22) | Tetrachloroethane (100) | 1.00,1,2-tetrachloroethane (10) | 50 | 1/2 hr. | DBA (3) | 110 | 8 hrs |
| 8 | 4,4′-MDA (1.22) | Octadecane (100) | 1.00 Toluene (20) | 30 | 1 hr. | DIPA (4.2) | 120 | 6 hrs. |
| 9 | 4,4′-MDA (1.22) | o-Dichlorobenzene (100) | 0.70 (as gas) | 50 | 1 hr. | DEA (3) | 140 | 3 hrs |

MDA - Methylene diphenylene diamine
PMPPA - Polymethylene polyphenylene polyamine
DEA - Diethylamine
DBA - Dibutylamine
DIPA - Diisopropylamine
DMA - Dimethylamine

TABLE 2

| Example No. | Promoter (g) | Temp. (° C) | Time (min) | Isocyanate Yield (%) |
|---|---|---|---|---|
| 5 | p-toluene $SO_3H$ (2.8) | 100 | 30 | 85 |
| 6 | "Nafion" (25) (as slurry) | 90 | 15 | 80 |
| 7 | N-ethylmorpholine hydrochloride (2) | 130 | 60 | 75 |
| 8 | 4,4′-dipyridyl dihydrochloride (3) | 150 | 30 | 70 |
| 9 | pyridine hydrobromide (2.0) | 120 | 60 | 89 |

## Claims

1. A process for the preparation of methylene diphenylene diisocyanates and polymethylene polyphenylene poly (isocyanates) which comprises the steps of

(a) reacting a methylene diphenylene diamine having the formula:

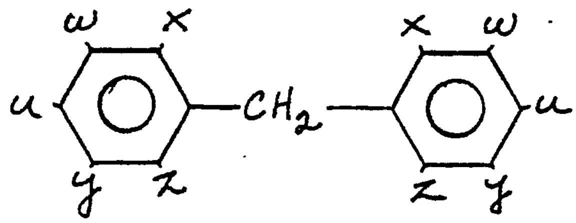

or a polymethylene polyphenylene polyamine having the formula:

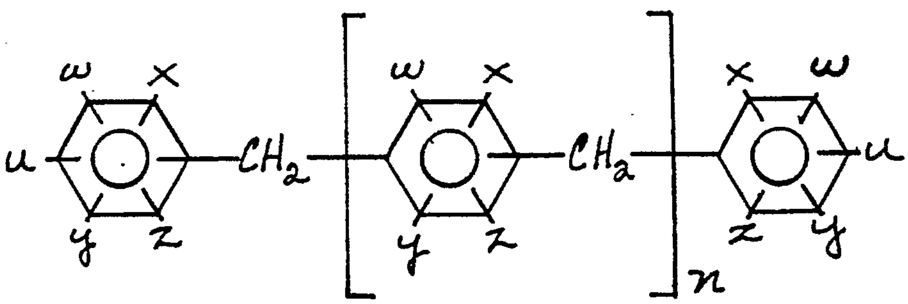

wherein n equals an integer of from 1 to 8 and at least one of the substituents u, w, x, y and z on the ring is an amino group and the other substituents which may be different on the ring are hydrogen, a 1 to 6 carbon alkyl group, a halogen, ether or nitro group, with isocyanic acid at a temperature of from about -30° C to about 200° C in the presence of an inert organic solvent to convert the amino groups to urea groups to produce a methylene diphenylene bis urea or a polymethylene polyphenylene polyurea;

(b) reacting said bis urea or polyurea thus produced with a dialkyl amine ($NHR'R''$) wherein $R'$ and $R''$ are substituted or unsubstituted mono-valent radicals selected from saturated or mono-olefinic unsaturated straight or branched chain aliphatic or cycloaliphatic radicals optionally containing alkoxyalkyl radicals with one or more ether linkages, aryl radicals, or aralkyl radicals, or $R'$ and $R''$ together form a substituted or unsubstituted saturated or mono-olefinic unsaturated divalent aliphatic radical, at a temperature of from about 50° C to about 200° C in an inert organic solvent to produce a methylene diphenylene bis (dialkyl urea) having the general formula:

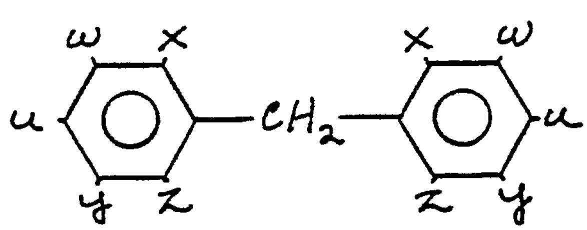

or a polymethylene polyphenylene poly (alkyl urea) having the formula:

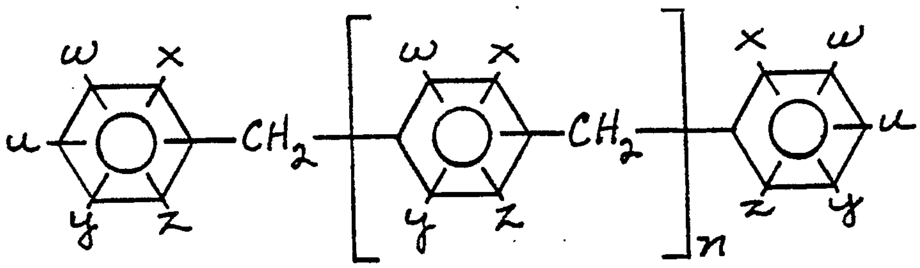

wherein n equals an integer of from 1 to 8 and at least one of the substituents u, w, x, y and z on the ring is a dialkylureido group having the formula (-NHCONR′R″) and the other substituents which may be different on the ring, are hydrogen, a 1 to 6 carbon alkyl group, a halogen, an ether group or a nitro group and R′ and R″ are as defined above

(c) thermally decomposing said methylene diphenylene bis (dialkylurea) or polymethylene polyphenylene poly (alkylurea) dissolved or slurried in an inert organic solvent or mixture of solvents, in the presence of a reaction promoter selected from tertiary amine hydrohalides, phosphorus pentoxide, organic sulfonic acids, and sulfonated aromatic ion exchange resins, at a temperature of from about 50°C to about 200°C; and

(d) recovering the methylene diphenylene diisocyanate or polymethylene polyphenylene poly (isocyanate).

2. A process according to Claim 1 wherein the temperature of reacting the diamine or polyamine with isocyanic acid is in the range of from about -10°C to 150°C.

3. A process according to Claim 1 or Claim 2 wherein the temperature of reacting the bis urea or polyurea with a dialkyl amine is in the range of from about 90°C to 150°C.

4. A process according to any one of Claims 1 to 3 wherein the temperature of thermally decomposing the bis (dialkylurea) or poly (alkylurea) is in the range of from about 90°C to 150°C.

5. A process for the preparation of methylene diphenylene diisocyanates which comprises the steps of

(a) reacting a methylene diphenylene diamine having the formula:

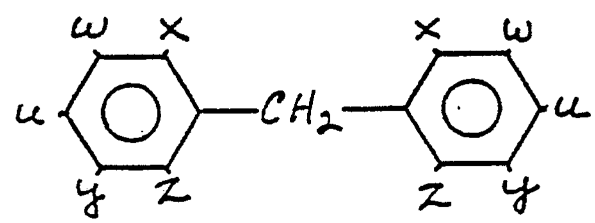

wherein at least one of the substituents u, w, x, y and z on the ring is an amine group and the other substituents which may be different on the ring are hydrogen, a 1 to 6 carbon alkyl group, a halogen, an ether or nitro group, with isocyanic acid at a temperature of from about -10° C to 150° C in the presence of an inert organic solvent to convert the amino groups to urea groups to produce a methylene diphenylene bis urea;

(b) reacting said bis urea or polyurea thus produced with a dialkyl amine (NHR′R″) wherein R′ and R″ are substituted or unsubstituted mono-valent radicals selected from saturated or mono-olefinic unsaturated straight or branched chain aliphatic or cycloaliphatic radicals optionally containing alkoxyalkyl radicals with one or more ether linkages, aryl radicals, or aralkyl radicals, or R′ and R″ together form a substituted or unsubstituted saturated or mono-olefinic unsaturated divalent aliphatic radical, at a temperature of from about 90° C to about 150° C in an inert organic solvent to produce a methylene diphenylene bis (dialkyl urea) having the formula:

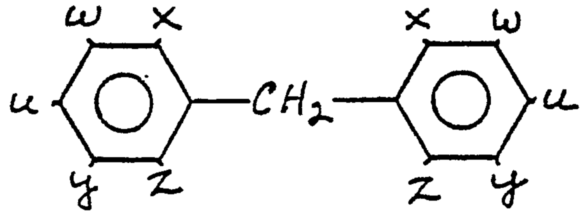

wherein at least one of the substituents u, w, x, y and z on the ring is a dialkylureido group having the formula (-NHCONR′R″) and the other substituents which may be different on the ring are hydrogen, a 1 to 6 carbon alkyl group, a halogen, an ether group or nitro group, and R′ and R″ are as defined above;

(c) thermally decomposing said methylene diphenylene bis (dialkylurea) dissolved in or slurried in an inert organic solvent or mixture of solvents in the presence of a reaction promoter selected from tertiary amine hydrohalides, phosphorus pentoxide, organic sulfonic acids and sulfonated aromatic ion exchange resins, at a temperature of from about 90 °C to 150 °C; and

(d) recovering the methylene diphenylene diisocyanate.

6. A process according to any one of Claims 1 to 5 wherein the diamine is selected from 4,4′-methylene diphenylene diamine and mixtures of 2,4′- and ,4,4′-methylene diphenylene diamine.

7. A process according to any one of Claims 1 to 6 wherein the dialykl amine reactant is selected from dimethylamine, diethylamine, dibutylamine and diisopropylamine.

8. A process according to any one of Claims 1 to 7 wherein the organic solvents employed in the steps of the process are selected from xylenes, o-dichlorobenzene, chlorobenzene, tetrachloroethane, nitrobenzene, diphenyl ether and octadecane.

9. A process according to any one of Claims 1 to 8 wherein the tertiary amine hydrohalide is selected from pyridine hydrochloride, pyridine hydrobromide N-ethylmorpholine hydrochloride, and 4,4′ dipyridyl dihydrochloride.

10. A process according to any one of Claims 1 to 9 wherein the organic sulfonic acid is selected from methane sulfonic acid and p-toluene sulfonic acid.

11. A process according to any one of Claims 1 to 9, wherein the sulfonated aromatic ion exchange resin is selected from sulfonated styrene/divinylbenzene copolymer resins and acidic perfluoroalkane sulfonic acid resins.

European Patent Office

# EUROPEAN SEARCH REPORT

Application Number

EP 90 30 5738

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-2 029 413 (AKZO)<br>* Claims; examples 5-11 * | 1-11 | C 07 C 265/14<br>C 07 C 263/06<br>C 07 C 275/28<br>C 07 C 273/18 |
| D,A | US-A-3 936 484 (R. ROSENTHAL)<br>* Claims; examples * | 1-11 | |
| A | FR-A-2 139 577 (QUIMCO)<br>* Claims; example 3 * | 1-11 | |
| D,A | FR-A-1 473 821 (SOCIETE CARBOCHIMIQUE)<br>* Whole document * | 1-11 | |
| D,A | US-A-2 773 086 (R.J. SLOCOMBE)<br>* Whole document * | 1-11 | |
| A | GB-A-1 154 278 (CIBA)<br>* Whole document * | 1-11 | |
| P,X | US-A-4 873 364 (E.T. SHAWL)<br>* Whole document * | 1-11 | TECHNICAL FIELDS SEARCHED (Int. Cl.5) |
| E | EP-A-0 371 698 (ARCO CHEMICAL TECHNOLOGY)<br>* Whole dcument * | 1-11 | C 07 C 263/00<br>C 07 C 265/00<br>C 07 C 273/00<br>C 07 C 275/00 |

The present search report has been drawn up for all claims

| Place of search | Date of completion of the search | Examiner |
|---|---|---|
| THE HAGUE | 13-09-1990 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document